# EUROPEAN PATENT APPLICATION

(11) **EP 1 604 993 A1**
(43) Date of publication of application: **14.12.2005**
(21) Application number: 04714951.3
(22) Date of filing: 26.02.2004
(51) Int. Cl.: C07D 519/00, A61K 31/4375, A61P 31/18, A61P 43/00, G01N 33/566, G01N 33/53

(54) **INHIBITORS**

(30) Priority: 28.02.2003 JP 2003054725
(71) Applicant: Japan Science and Technology Agency, Kawaguchi-shi, Saitama 332-0012 (JP)
(72) Inventor: NAKATANI, Kazuhiko, Kyoto 611-0013 (JP); HORIE, Souta, Kyoto 606-8225 (JP); SAITO, Isao, Kyoto 607-8242 (JP); HAGIWARA, Shinya, 2-1, Hirosawa Wako-shi, Saitama 351-0198 (JP); GOTO, Yuki, Kyoto 615-0907 (JP); KOBORI, Akio, Ktoto 606-8175 (JP)
(74) Representative: Bentz, Jean-Paul
(86) International application number: PCT/JP2004/002314
(87) International publication number: WO 2004/076463

(57) **Abstract**

An inhibitor contains naphthyridine dimer, a naphthyridine-azaquinolone hybrid, a trinaphthyridine-azaquinolone hybrid, or a trinaphthyridine-azaquinolone hybrid derivative. In order to inhibit binding of 100 nM of RRE to 100 nM of Rev protein, for example, an inhibitor containing naphthyridine dimer is used at a molarity of 1.2 µM to 12 µM, an inhibitor containing the naphthyridine-azaquinolone hybrid is used at a molarity of 2 µM to 20 µM, or an inhibitor containing the trinaphthyridine-azaquinolone hybrid derivative is used at a molarity of 200 nM to 2 µM. This makes it possible to effectively inhibit binding of RRE to Rev protein.

## Description

### TECHNICAL FIELD

The present invention relates to an inhibitor for inhibiting binding of RRE to Rev protein.

### BACKGROUND ART

There have been made continuous efforts to develop drugs for inhibiting infection and/or development of HIV-1, which is a causative pathogen of AIDS.

HIV-1 is a type of retrovirus, and when HIV-1 infects a normal cell, the HIV-1 introduces its mRNA into the normal cell. From the mRNA, a reverse transcriptase forms virus cDNA, which binds to a chromosome gene in a nucleus of the cell. Gene transcription for proliferation of the cell is accompanied with transcription of RNA with a gene of the virus.

Normally, RNA is spliced so that a mature mRNA is discharged out of a nucleus. However, to be discharged out of the nucleus, the HIV-1 RNA must not be spliced. What is important in this mechanism is an interaction between RRE (Rev Responsible Element) of the HIV-1 mRNA and Rev protein. Fig. 1 illustrates a base sequence of RRE. Fig. 2 illustrates a structure of Rev protein. Fig. 3 schematically illustrates how HIV-1 duplicates as a result of the binding of RRE to Rev protein.

Inhibition of the binding (interaction) of RRE to Rev protein causes the splicing of the HIV-1 mRNA. The splicing shortens the HIV-1 mRNA, so that the virus cannot proliferate. Therefore, a substance that inhibits the binding of RRE to Rev protein is considered as having an effect of suppressing development of AIDS.

So far some substances have been reported as inhibitors which inhibit the binding of RRE to Rev protein. Exemplified as one of such substances serving as the inhibitors is neomycine B, which is a natural antibiotic. The neomycine B is represented by the following structural formula ①. The neomycine B firmly binds to RRE, so that RRE cannot bind to Rev protein.

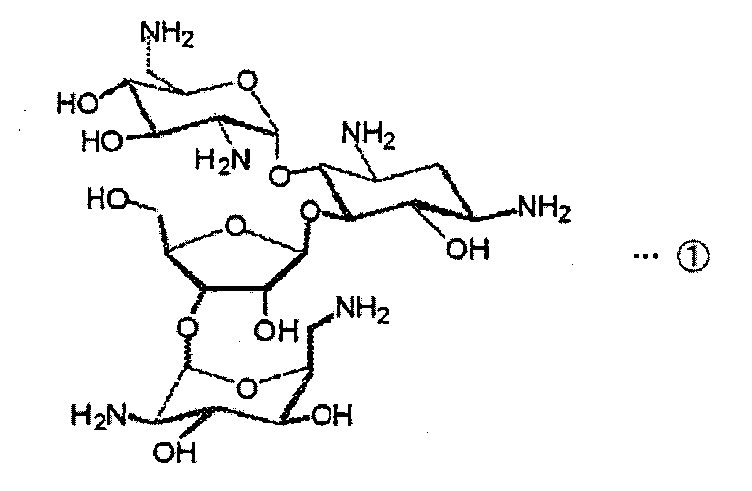

There are some artificially synthesized substances each of which can bind to RRE, and they are disclosed for example in Luedtke et al. Journal of American Chemical Society, 122, 980 (2000) (hereinafter referred to as "Non-patent Document 1") and Wilson et al. Biochemistry 40, 1150 (2001) (hereinafter referred to as "Non-patent Document 2"). The substance disclosed in Non-patent Document 1 is represented by the following structural formula ② , and the substance disclosed in Non-patent Document 2 is represented by the following structural formula ③.

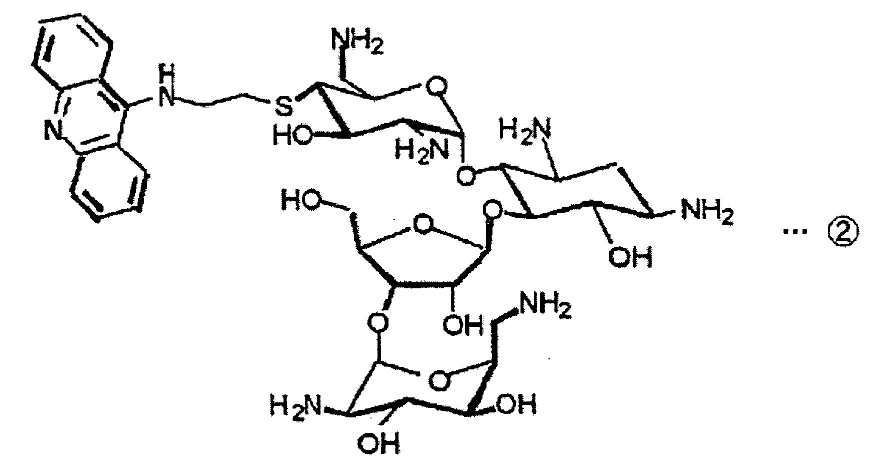

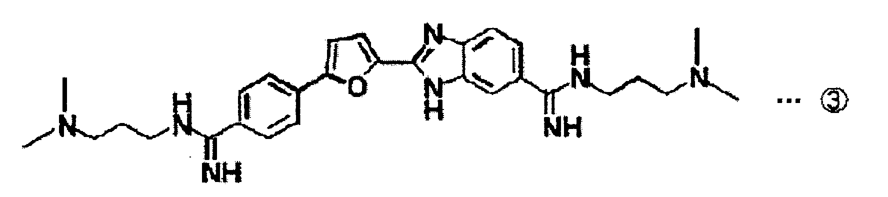

However, most of the substances exemplified as the conventional inhibitors are aminoglycoside obtained by screening natural products. Further, each of the substances capable of serving as the conventional inhibitors does not have a structure designed to specifically bind to a Rev protein binding site of RRE. Therefore, nonspecific binding of the substance is strong and the substance does not have a sufficient inhibitory activity.

Accordingly, it is an object of the present invention to provide an inhibitor which has a sufficient inhibitory activity and firmly binds to a Rev protein binding site of RRE.

### DISCLOSURE OF INVENTION

The inventors have found so far that naphthyridine dimer, which includes two naphthyridines each having a hydrogen-binding moiety complementary to a guanine, selectively binds to a guanine-guanine mismatch (hereinafter referred to as "GG mismatch") base pair of double-stranded DNA. The inventors have also found that a naphthyridine-azaquinolone hybrid, which is obtained by replacing one of the two naphthyridines of naphthyridine dimer with an azaquinolone having a hydrogen-binding moiety complementary to an adenine, selectively binds to a guanine-adenine mismatch (hereinafter referred to as "GA mismatch") base pair.

In devising the present invention, the inventors focused attention on the fact that an internal loop of RRE has a GG mismatch base pair and a GA mismatch base pair. The inventors have invented a novel inhibitor which inhibits binding of RRE to Rev protein by specifically binding to a Rev protein binding site of RRE.

Specifically, an inhibitor according to the present invention is an inhibitor for inhibiting binding of RRE to Rev protein, the inhibitor containing a naphthyridine derivative represented by general formula (A): 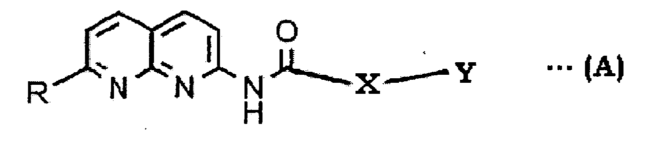 where R and R₁ are mutually independently a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms respectively substituted by an oxygen atom or a nitrogen atom, or a mono- or di-alkylamino group of a carbon number of 1 to 15, which is unsubstituted or has one or more carbon atoms substituted respectively by an oxygen atom or a nitrogen atom; X is an arbitrary bivalent group; and Y is a substituent represented by general formula (B) or a substituent represented by general formula (C): 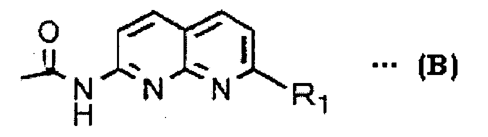 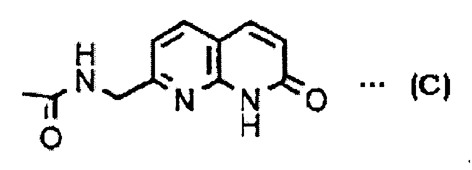 Further, a naphthyridine derivative according to the present invention is a naphthyridine derivative represented by general formula (A).

The naphthyridine derivative contains not only a single naphthyridine moiety but also at least either a naphthyridine moiety represented by general formula (B) or an azaquinolone moiety represented by general formula (C) so as to specifically bind to a GG mismatch base pair in an internal loop of RRE. According to this arrangement, it is possible to provide an inhibitor which can inhibit binding of RRE to Rev protein by binding to RRE.

The inhibitor according to the present invention is an inhibitor for inhibiting binding of RRE to Rev protein and contains naphthyridine dimer represented by general formula (1): 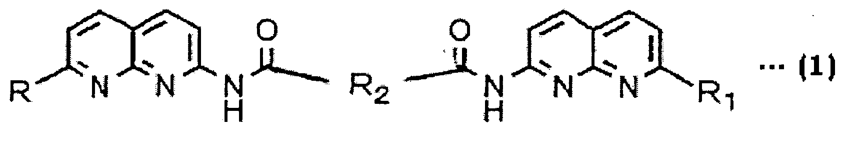 where R and R₁ are mutually independently a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom, and R₂ is an alkylene group of a carbon number of 1 to 20, which is unsbstituted or has one or more carbon atoms respectively substituted by an oxygen atom, a sulfur atom, a nitrogen atom, or a carbonyl group.

The inhibitor according to the present invention is arranged so as to contain a naphthyridine-azaquinolone hybrid represented by general formula (2): 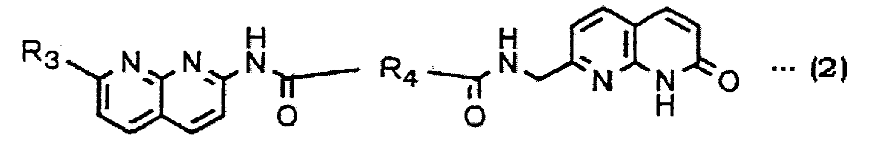 where R₃ is a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom, and R₄ is an alkylene group of a carbon number of 1 to 20, which is unsbstituted or has one or more carbon atoms respectively substituted by an oxygen atom, a sulfur atom, a nitrogen atom, or a carbonyl group.

The inhibitor contains naphthyridine dimer so as to specifically bind to a GG mismatch base pair in an internal loop of RRE. Further, the inhibitor contains the naphthyridine-azaquinolone hybrid so as to specifically bind to a GA mismatch base pair in an internal loop of RRE. According to this arrangement, it is possible to provide an inhibitor which can inhibit the binding of RRE to Rev protein by binding to RRE.

The inhibitor according to the present invention is arranged so as to contain a trinaphthyridine-azaquinolone hybrid represented by general formula (3): 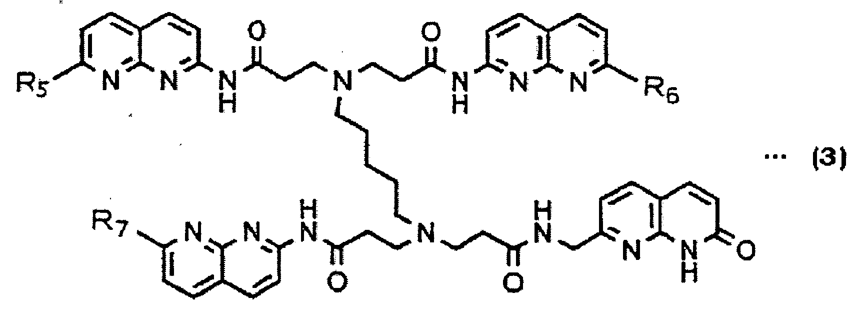 where R₅ ,R₆, and R₇ are mutually independently a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom.

As shown in general formula (3), the trinaphthyridine-azaquinolone hybrid contains two trinaphthyridines and one azaquinolone. Therefore, an inhibitor containing the trinaphthyridine-azaquinolone hybrid specifically binds simultaneously to a GG mismatch base pair and a GA mismatch base pair in an internal loop of RRE. According to this arrangement, it is possible to provide an inhibitor which can more effectively inhibit binding of RRE to Rev protein by binding to RRE.

The inhibitor according to the present invention which contains the trinaphthyridine-azaquinolone hybrid can be obtained for example by binding a naphthyridine dimer to an azaquinolone hybrid by using a linker. The inhibitor according to the present invention may be arranged so as to contain a trinaphthyridine-azaquinolone hybrid derivative which is obtained by appropriately adjusting a structure of the linker and is represented by general formula (4): 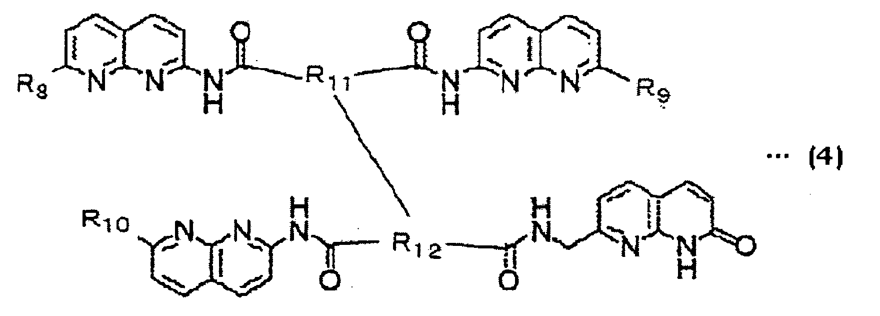 where R₈, R₉, and R₁₀ are mutually independently a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom, and R₁₁ and R₁₂ are mutually independently an alkylene group of a carbon number of 1 to 20, which is unsubstituted or has one or more carbon atoms substituted respectively by an oxygen atom, a sulfur atom, a nitrogen atom, or a carbonyl group. The inhibitor containing such a trinaphthyridine-azaquinolone hybrid derivative can also effectively inhibit binding of RRE to Rev protein.

The inhibitor according to the present invention which contains the trinaphthyridine-azaquinolone hybrid derivative can be obtained through a step of synthesizing as an intermediate an aldehyde by allowing naphthyridine dimer or a naphthyridine-azaquinolone hybrid to react with a di-aldehyde represented by general formula (D):

OHC-R_{A}-CHO (D)

where R_{A} is an alkylene group of a carbon number of 1 to 13, which is unsubstituted or has one or more carbon atoms substituted respectively by an oxygen atom or a nitrogen atom. In particular, the alkylene group indicated by R_{A} is an alkylene group having 1 to 3 carbon atoms. The aldehyde thus obtained is a naphthyridine-dimer-containing aldehyde represented by general formula (5): 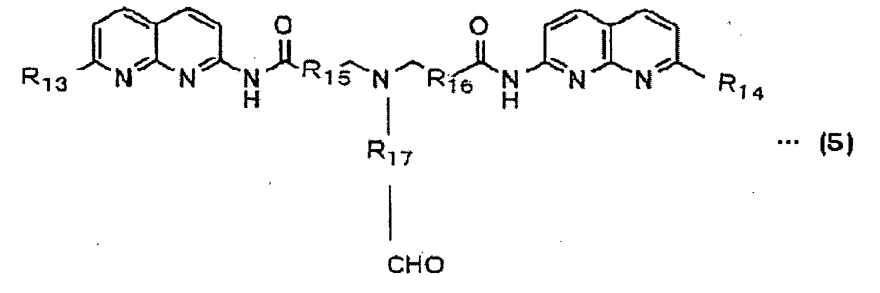 where R₁₃, and R₁₄ are mutually independently a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom; R₁₅ and R₁₆ are mutually independently an alkylene group of a carbon number of 1 to 18, which is unsubstituted or has one or more carbon atoms substituted respectively by an oxygen atom, a sulfur atom, a nitrogen atom, or a carbonyl group; and R₁₇ is an alkylene group of a carbon number of 1 to 14, which is unsubstituted or has one or more carbon atoms substituted respectively by an oxygen atom or a nitrogen atom.
Alternatively, the aldehyde thus obtained is a naphthyridine-azaquinolone-hybrid-containing aldehyde represented by general formula (6): 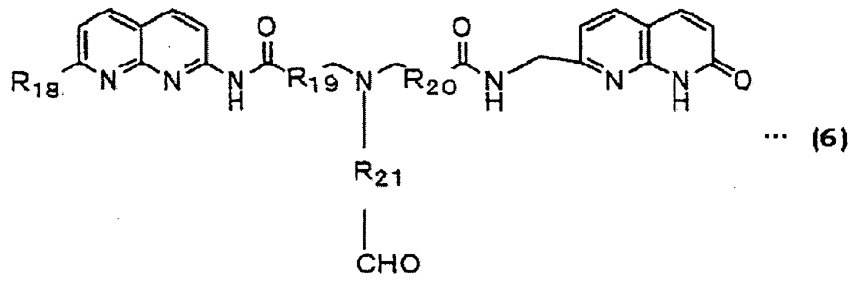 where R₁₈ is a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom; R₁₉ and R₂₀ are mutually independently an alkylene group of a carbon number of 1 to 18, which is unsubstituted or has one or more carbon atoms substituted respectively by an oxygen atom, a sulfur atom, a nitrogen atom, or a carbonyl group; and R₂₁ is an alkylene group of a carbon number of 1 to 14, which is unsubstituted or has one or more carbon atoms substituted respectively by an oxygen atom or a nitrogen atom.
In particular, the alkylene group indicated by each of R₁₇ and R₂₁ is an alkylene group having 1 to 4 carbon atoms.

Further, the present invention provides a substrate including a composite which contains at least one compound selected from the compound group consisting of naphthyridine dimer, the naphthyridine-azaquinolone hybrid, and the trinaphthyridine-azaquinolone hybrid or a derivative thereof.

Such a substrate can be used to arrange an instrument such as a chip, a cuvette, or a plate which has a function of detecting RRE in a culture medium or a blood fluid.

Further, a biosensor according to the present invention includes a naphthyridine derivative according to the present invention immobilized on a carrier such as a chip, a cuvette, or a plate.

According to the biosensor, it is possible to detect RRE contained in a culture medium or a blood fluid, so that infection of HIV-1 and other conditions can be detected.

For a fuller understanding of the nature and advantages of the invention, reference should be made to the ensuing detailed description taken in conjunction with the accompanying drawings.

### BRIEF DESCRIPTION OF DRAWINGS

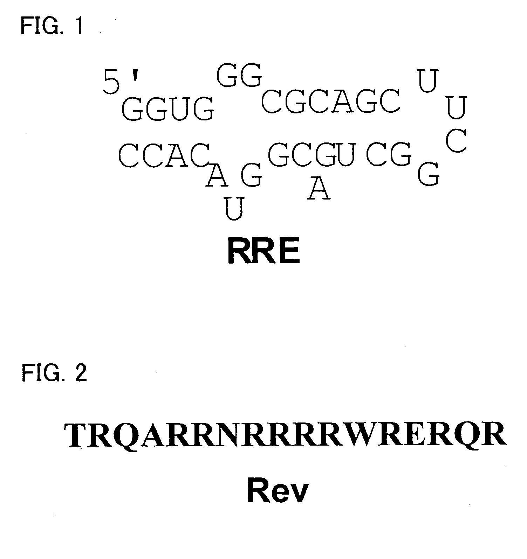

Fig. 1 illustrates a base sequence of RRE.
Fig. 2 illustrates a structure of Rev protein.

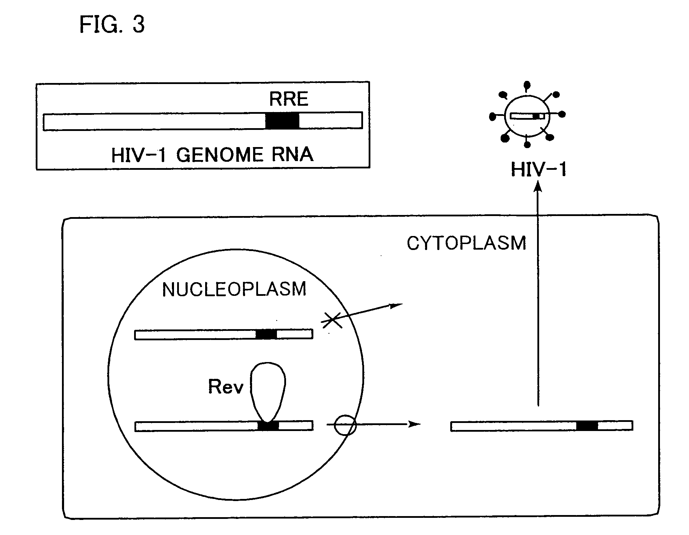

Fig. 3 schematically illustrates how HIV-1 is replicated due to binding of RRE to Rev protein.

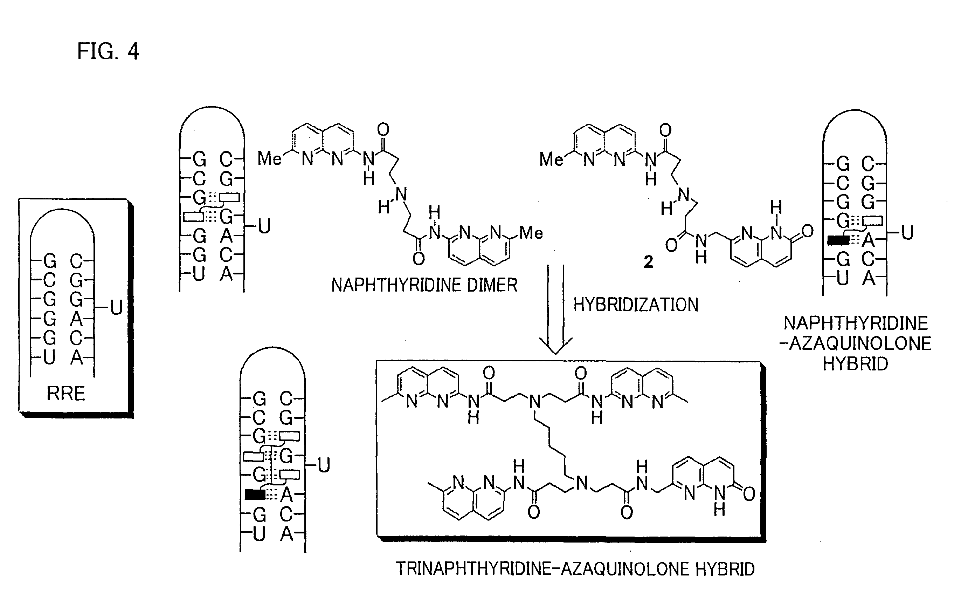

Fig. 4 is schematically illustrates how naphthyridine dimer and a naphthyridine-azaquinolone hybrid are bound to each other by using a linker.

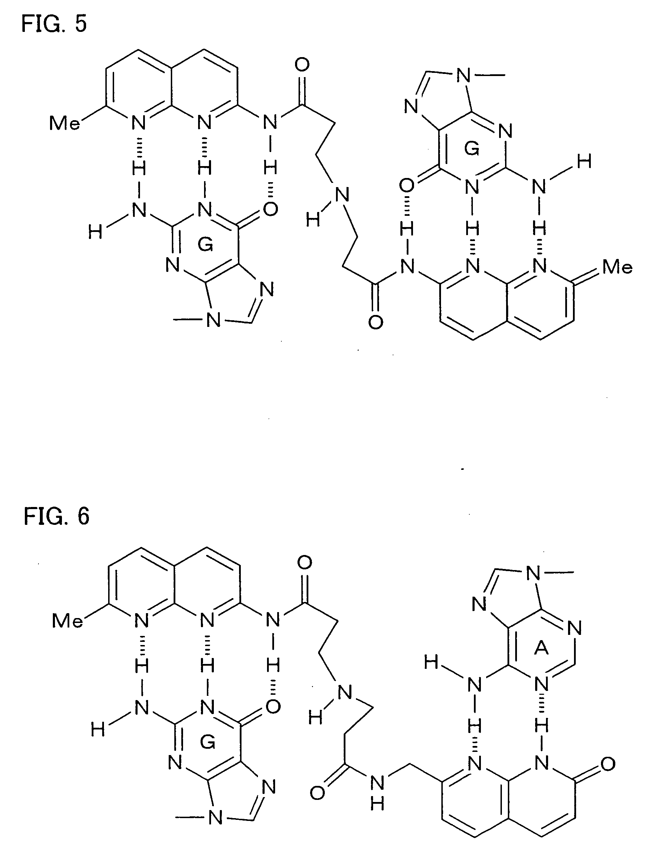

Fig. 5 illustrates how naphthyridine dimer serving as an inhibitor is bound to a GG mismatch base pair in an internal loop of RRE.
Fig. 6 illustrates how the naphthyridine-azaquinolone hybrid serving as an inhibitor is bound to the GA mismatch base pair in the internal loop of RRE.

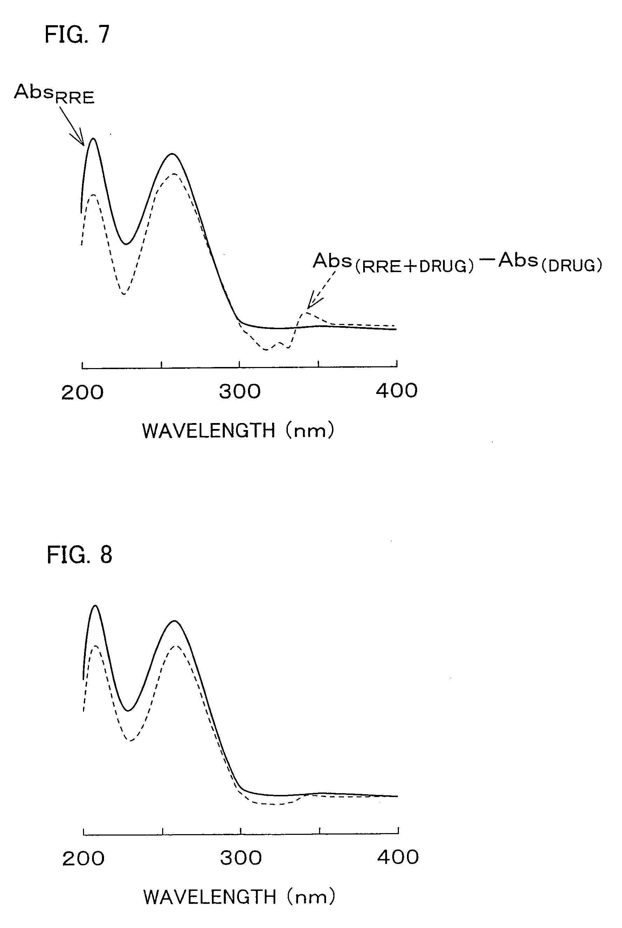

Fig. 7 illustrates a UV absorption spectrum of a mixture of naphthyridine dimer and RRE in Example 2.
Fig. 8 illustrates a UV absorption spectrum of a mixture of the naphthyridine-azaquinolone hybrid and RRE in Example 2.

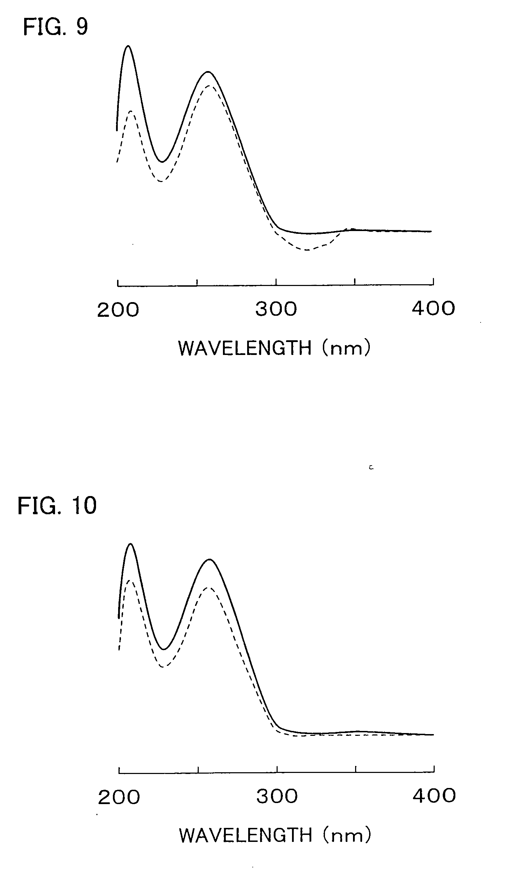

Fig. 9 illustrates a UV absorption spectrum of a mixture of a primary quinoline-containing compound and RRE in Example 2.
Fig. 10 illustrates a UV absorption spectrum of a mixture of a secondary quinoline-containing compound and RRE in Example 2.

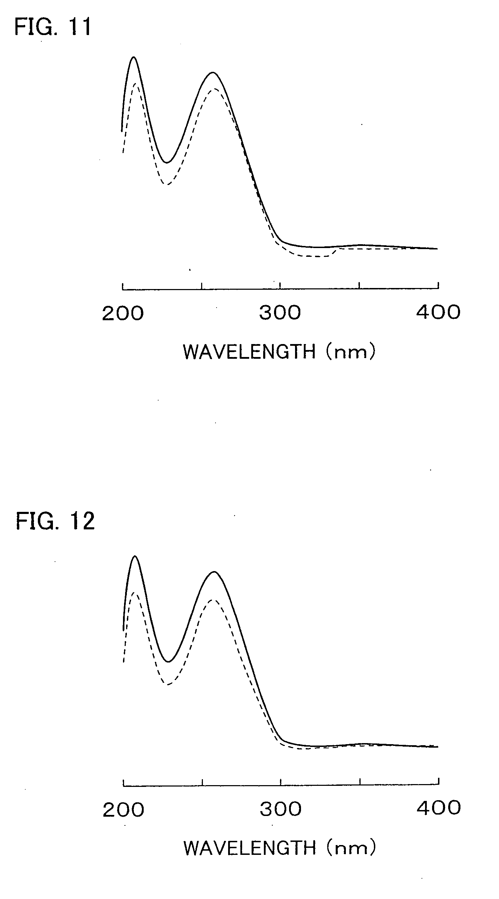

Fig. 11 illustrates a UV absorption spectrum of a mixture of naphthyridine and RRE in Example 2.
Fig. 12 illustrates a UV absorption spectrum of a mixture of azaquinolone and RRE in Example 2.

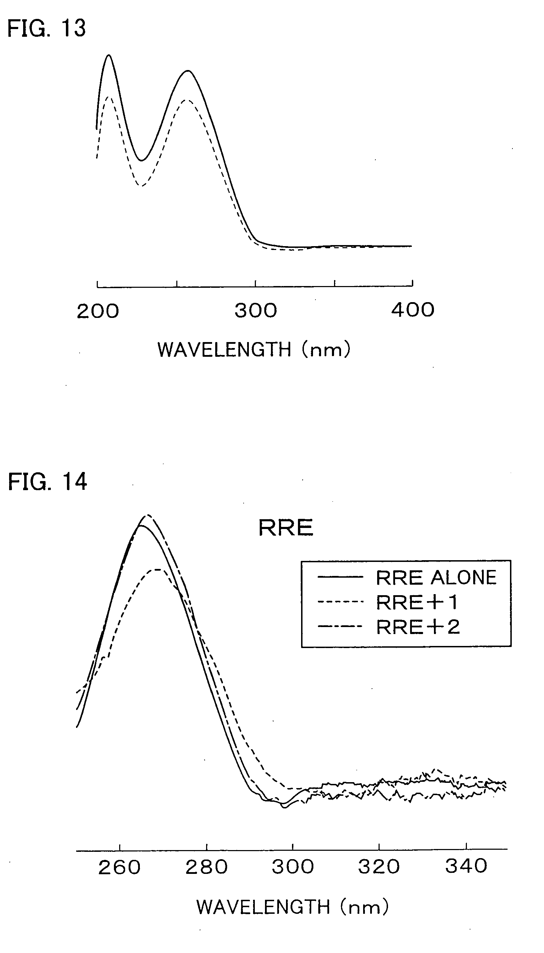

Fig. 13 illustrates a UV absorption spectrum of a mixture of quinolone and RRE in Example 2.
Fig. 14 shows a CD spectrum of RRE alone in Example 3, a CD spectrum of a mixture of naphthyridine dimer and RRE in Example 3, and a CD spectrum of a mixture of the naphthyridine-azaquinolone hybrid and RRE in Example 3.

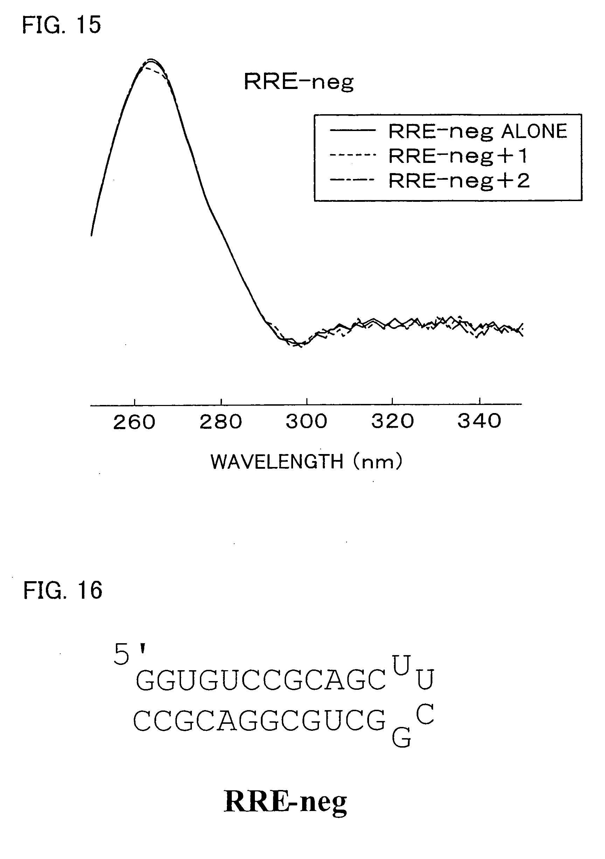

Fig. 15 illustrates a CD spectrum of RRE-neg alone in Example 3, a CD spectrum of a mixture of naphthyridine dimer and RRE-neg in Example 3, and a CD spectrum of a mixture of the naphthyridine-azaquinolone hybrid and RRE-neg in Example 3.
Fig. 16 illustrates a base sequence of RRE-neg in Example 3.

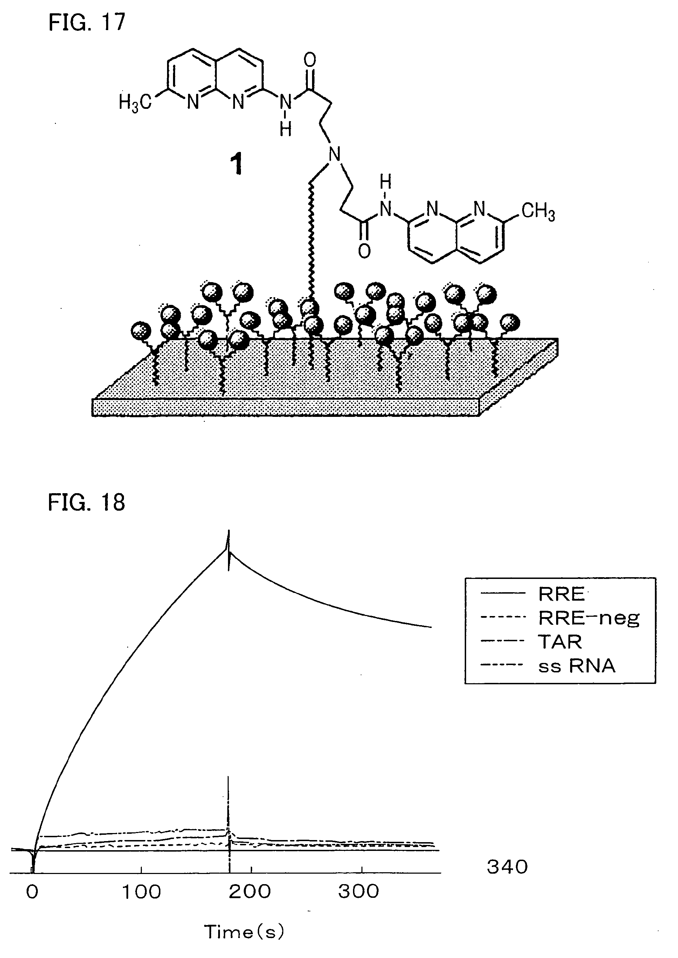

Fig. 17 schematically illustrates how to study respective bindings of RRE, RRE-neg, TAR, and single-stranded RNA to naphthyridine dimer immobilized on an SPR sensor chip in Example 4.
Fig. 18 schematically illustrates strengths of the respective bindings of RRE, RRE-neg, TAR, and single-stranded RNA to naphthyridine dimer immobilized on the SPR sensor chip in Example 4.

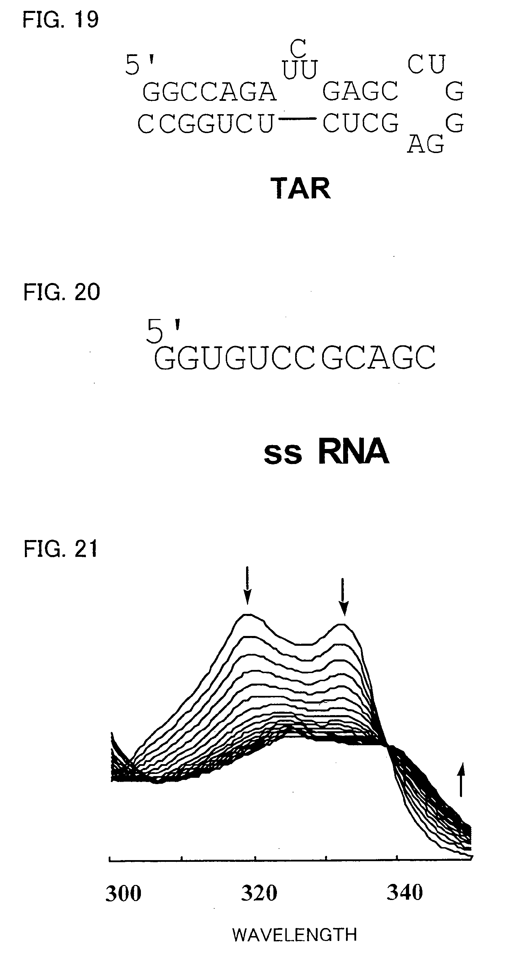

Fig. 19 illustrates a base sequence of TAR in Example 4.
Fig. 20 illustrates a base sequence of a single-stranded RNA in Example 4.
Fig. 21 illustrates UV absorption spectra of naphthyridine dimer in the presence of RRE of various concentrations in Example 5.

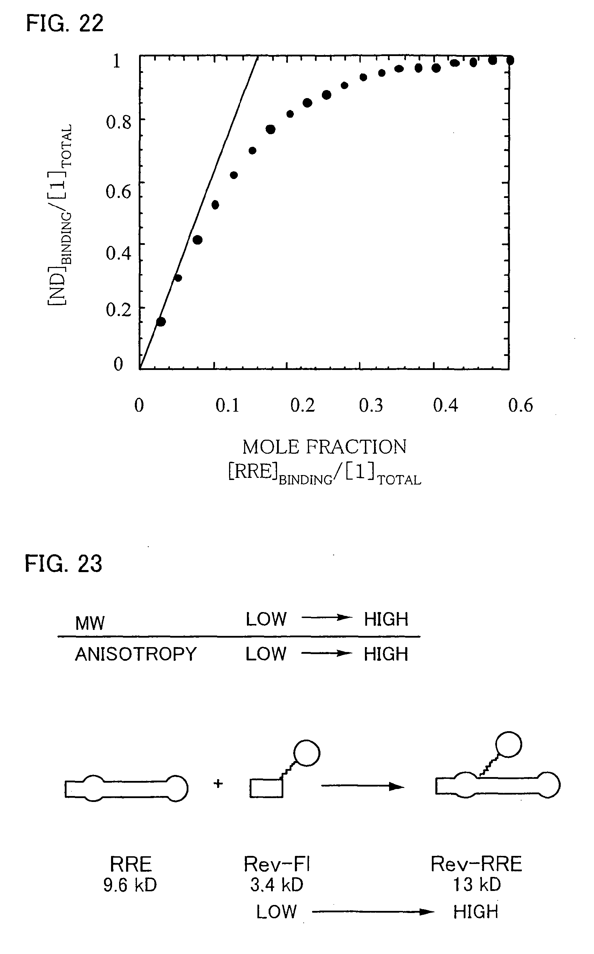

Fig. 22 illustrates a proportion of naphthyridine dimer binding to RRE to the whole naphthyridine dimer added to RRE, the proportion being calculated from absorption intensity at 332 nm of Fig. 21.
Fig. 23 schematically illustrates how a complex of Rev-Fl fluorescent labeled with fluorescein and RRE was formed in Example 6.

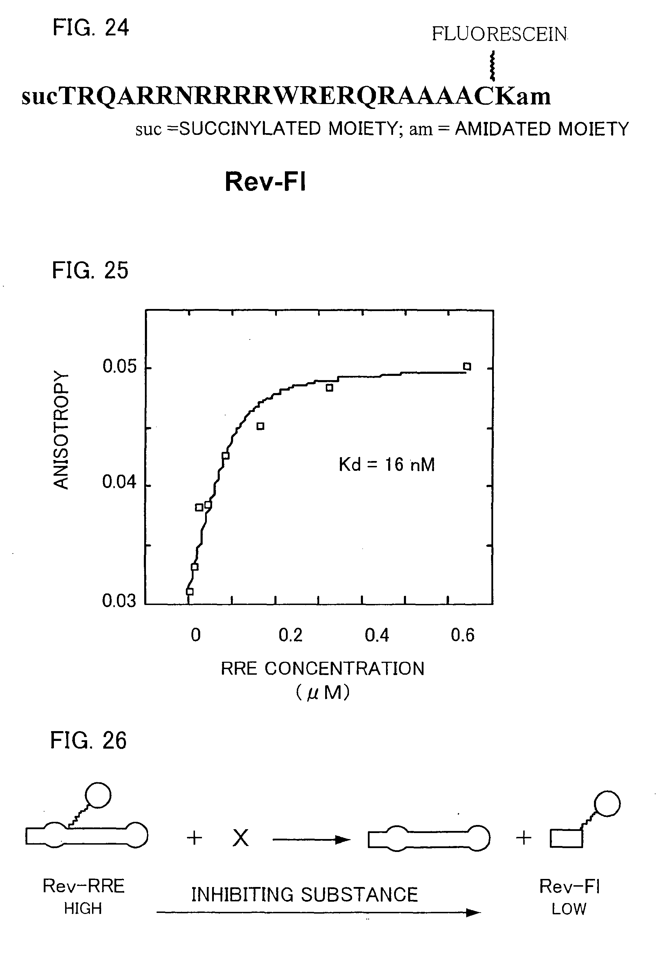

Fig. 24 illustrates a structure of the Rev-Fl in Example 6.
Fig. 25 illustrates a change in anisotropy of the Rev-Fl in the presence of RRE of various concentrations in Example 6.
Fig. 26 illustrates the anisotropy in the presence of respective inhibitors of various concentrations. The inhibitors were naphthyridine dimer, the naphthyridine-azaquinolone hybrid, and neomycine B.

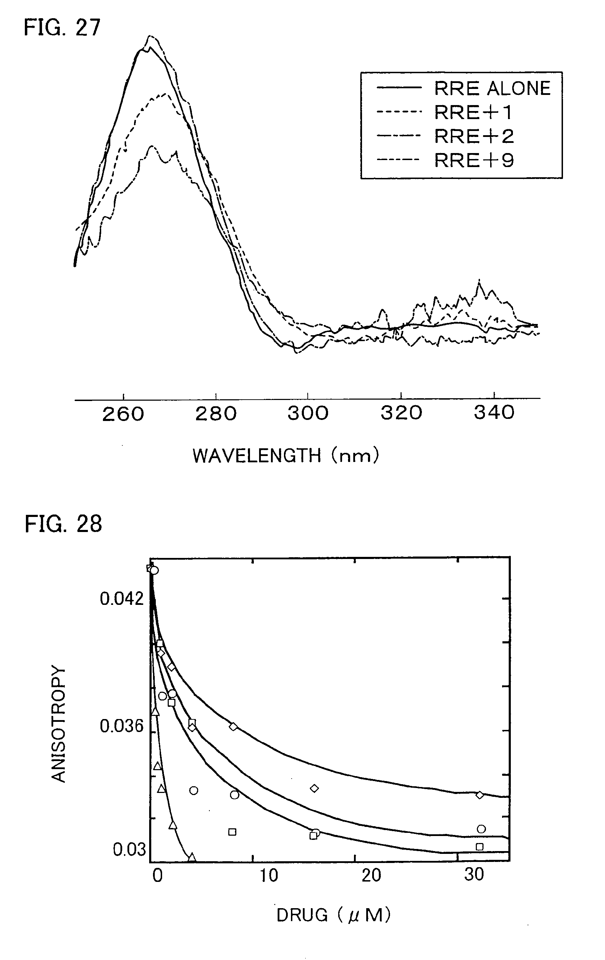

Fig. 27 illustrates how CD spectra of RRE were changed in Example 8, in the following cases: RRE alone; when naphthyridine dimer was added to RRE; when the trinaphthyridine-azaquinolone hybrid was added to RRE; and when the trinaphthyridine-azaquinolone hybrid was added to RRE .
Fig. 28 illustrates anisotropies measured in cases where naphthyridine dimer, the naphthyridine-azaquinolone hybrid, the neomycine B, and the triazaquinolone hybrid were respectively used as the inhibitors.

### BEST MODE FOR CARRYING OUT THE INVENTION

One embodiment of the present invention will be described below. An inhibitor according to the present invention is arranged so as to contain naphthyridine dimer represented by the foregoing general formula (1).

Naphthyridine dimer according to the present embodiment is not particularly limited. Therefore, naphthyridine dimer may be synthesized by various conventional methods. For example, naphthyridine dimer represented by general formula (1) can be obtained by bonding two molecules of 1,8-naphthyridine derivative(s) with a linker.

The 1,8-naphthyridine derivative(s) may be obtained as follows. For example, 2-amino-1,8-naphthyridine or 2-amino-7-methyl-1,8-naphthyridine is allowed to react with a reactive derivative of N-protection-4-amino butyric acid, e.g., an acid chloride so as to acylate the amino group at the second position. Thereafter, the amino group is deprotected by removing the protecting group. In this way, the 1,8-naphthyridine derivative is obtained.

The inhibitor according to the present embodiment may be arranged so as to contain a naphthyridine-azaquinolone hybrid which is represented by the foregoing general formula (2) and is such a compound that one naphthyridine of naphthyridine dimer represented by general formula (1) is replaced with azaquinolone.

The naphthyridine-azaquinolone hybrid according to the present embodiment is not particularly limited. Therefore, the naphthyridine-azaquinolone hybrid may be synthesized by various conventional methods.

The inhibitor according to the present embodiment may contain a trinaphthyridine-azaquinolone hybrid represented by the foregoing general formula (3).

As shown in Fig. 4, the trinaphthyridine-azaquinolone hybrid may be obtained by bonding naphthyridine dimer to the naphthyridine-azaquinolone hybrid by using a linker. The linker is not particularly limited but may be appropriately adopted according to need. Further, the inhibitor according to the present embodiment may contain a trinaphthyridine-azaquinolone hybrid derivative represented by the foregoing formula (4), by appropriately adjusting a structure of the linker.

The trinaphthyridine-azaquinolone hybrid can be obtained as follows. For example, the trinaphthyridine-azaquinolone hybrid is allowed to react with naphthyridine dimer and a dialdehyde such as a glutaric aldehyde, thereby to synthesize, as an intermediate, a naphthyridine-dimer-containing aldehyde represented by the foregoing general formula (5). Naphthyridine dimer-containing aldehyde is allowed to react with the naphthyridine-azaquinolone hybrid, thereby to obtain trinaphthyridine-azaquinolone.

Alternatively, the trinaphthyridine-azaquinolone hybrid can be obtained as follows. The naphthyridine-azaquinolone hybrid is allowed to react with a dialdehyde such as a glutaric aldehyde, thereby to synthesize, as an intermediate, a naphthyridine-azaquinolone-hybrid-containing aldehyde represented by the foregoing general formula (6). The naphthyridine-azaquinolone-hybrid-containing aldehyde is allowed to react with naphthyridine dimer, thereby to obtain the trinaphthyridine-azaquinolone hybrid.

Thus, the inhibitor according to the present embodiment contains naphthyridine dimer and/or the naphthyridine-azaquinolone hybrid. Therefore, the inhibitor has a property to be specifically bonded to a GG mismatch base pair in an internal loop of RRE and/or a GA mismatch base pair in the internal loop of RRE.

Fig. 5 shows how naphthyridine dimer serving as the inhibitor according to the present embodiment is bonded to the GG mismatch base pair in the internal loop of RRE. Fig. 6 shows how the naphthyridine-azaquinolone hybrid serving as the inhibitor according to the present embodiment is bonded to the GA mismatch base pair in the internal loop of RRE.

In the arrangement in which the inhibitor according to the present embodiment comprises naphthyridine dimer, it is preferable that the inhibitor be used in such a range that the inhibitor is of a molarity of 1.2 µM to 12 µM with respect to binding of 100 nM of RRE to 100 nM of Rev protein.

Further, in the arrangement in which the inhibitor comprises the naphthyridine-azaquinolone hybrid, it is preferable that the inhibitor be used in such a range that the inhibitor is of a morality of 2 µM to 20 µM M with respect to binding of 100 nM of RRE to 100 nM of Rev protein.

Further, in the arrangement in which the inhibitor comprises the trinaphthyridine-azaquinolone hybrid or a derivative of the trinaphthyridine-azaquinolone hybrid, it is preferable that the inhibitor be used in such a range that the inhibitor is of a morality of 200 nM to 2 µM with respect to binding of 100 nM of RRE to 100 nM of Rev protein.

The inhibitor according to the present embodiment specifically binds to RRE and can thereby inhibit genome RNA from being transported in a cytoplasm. Therefore, the inhibitor according to the present embodiment can be used as an anti-HIV-1 drug for inhibiting replication of the virus.

For example, a substrate (carrier) may be made from a composite which comprises at least one selected from the compound group consisting of naphthyridine dimer, the naphthyridine-azaquinolone hybrid, and the trinaphthyridine-azaquinolone hybrid and a derivatives thereof according to the present embodiment. Use of such a substrate can give, to an instrument such as a chip, a cuvette, or a plate, a function of detecting RRE in a culture medium or a blood fluid.

The present invention will be described below in detail by way of examples but is not to be limited in any way by these examples.

### [Example 1]

A trinaphthyridine-azaquinolone hybrid was synthesized as follows.
(i) Several drops of acetic acid were dripped into a methanol solution of naphthyridine dimer (73 mg) so that the solution had pH 5. To the solution, glutaric aldehyde (360 mg) and then sodium cyanoboron hydride (3.6 mg) were added. The resulting solution was allowed to react for 10 minutes at room temperature.
   The reaction mixture was distributed by a water-chloroform solution, and the organic layer was dehydrated and then concentrated. The crude product was purified by using preparative TLC, thereby to obtain naphthyridine-dimer-containing aldehyde (27.0 mg).
   Naphthyridine dimer-containing aldehyde thus obtained was analyzed by NMR. Results were as follows.
   ¹HNMR (CDCl₃, 400 MHz) δ=10.29 (br, 2H), 9.65 (br, 1H), 8.32 (d, 2H, J=8.8 Hz), 7.93 (d, 2H, J=9.2 Hz), 7.83 (d, 2H, J=8.0 Hz), 7.14 (d, 2H, J=8.4 Hz), 2.98 (m, 4H), 2.80 (br, 4H), 2.69 (s, 6H), 2.65 (m, 4H), 1.64 (m, 4H);
   ¹³CNMR (CDCl₃, 100 MHz) δ=202.6, 171.8, 162.7, 154.3, 153.6, 138.5, 136.2, 121.17, 118.2, 114.6, 53.7, 49.6, 43.5, 34.8, 25.7, 25.4, 19.9.
(ii) Several drops of acetic acid were dripped into a methanol solution of naphthyridine dimer-containing aldehyde (22.3 mg) so that the solution had pH 5. To the solution, naphthyridine azaquinolone (29.6 mg) and then sodium cyanoboron hydride (3.6 mg) were added. The resulting solution was allowed to react for 15 minutes at room temperature.

The reaction mixture was distributed by a water-chloroform solution, and the organic layer was dehydrated and then concentrated. The crude product was purified by using silica gel column chromatography, thereby to obtain trinaphthyridine azaquinolone (10.0 mg).

The foregoing synthetic reaction is represented below by formula A:

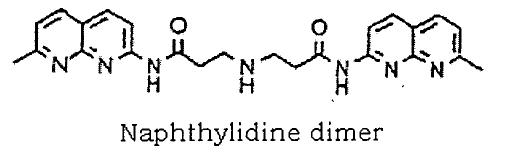

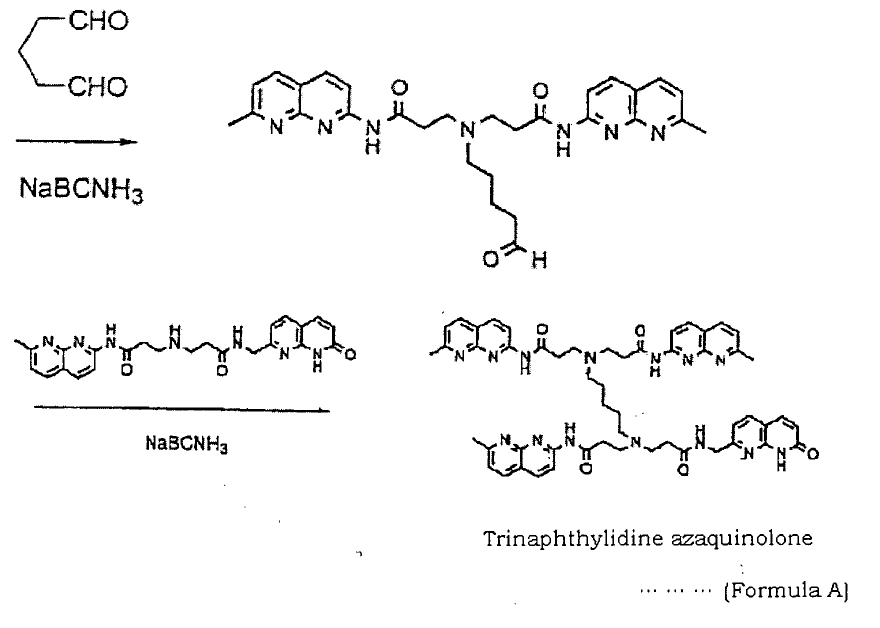

Trinaphthyridine azaquinolone thus obtained was analyzed by NMR. Results were as follows.

¹HNMR (CD₃OD, 400 MHz) δ=8.11 (br, 1H), 8.10 (d, 2H, J=8.8 Hz), 8.01 (d, 2H, J=8.4 Hz), 7.99 (d, 1H, J=8.0 Hz), 7.87 (d, 2H, J=8.4 Hz), 7.85 (d, 2H, J=8.4 Hz), 7.63 (d, 1H, J=8.0 Jz, 7.57 (d, 1H, J=9.6 Hz), 7.24 (d, 1H, J=8.4 Hz), 7.18 (d, 2H, J=8.4 Hz), 6.97 (d, 1H, J=7.6 Hz), 6.38 (d, 1H, J=9.6 Hz), 4.39 (s, 2H), 2.98 (br, 8H), 2.72 (10H), 2.62 (s, 3H), 2.61 (s, 6H), 2.60 (2H), 1.70 (m, 4H), 1.47 (m, 2H);
¹³CNMR (CD₃OD, 100 MHz) δ=173.7, 172.6, 164.5, 163.0, 162.7, 160.0, 153.9, 153.83, 153.78, 153.7, 148.5, 139.7, 138.9, 138.6, 137.4, 137.2, 136.7, 121.6, 121.3, 121.2, 118.5, 118.4, 116.1, 114.4, 114.3, 113.3, 54.05, 53.8, 50.3, 49.6, 49.4, 48.7, 44.6, 33.9, 25.9, 24.9, 23.9, 23.8.

### [Example 2]

Referring to UV absorption spectra of RRE in the presence and absence of naphthyridine dimer, screenings of the drugs was carried out. The results of the screenings are shown in Figs. 7 to 10.

Fig. 7 shows a UV absorption spectrum of a mixture of naphthyridine dimer and RRE. In Fig. 7, the solid line indicates the UV absorption spectrum of RRE alone, and the dotted line indicates a UV absorption spectrum when the absorption of naphthyridine dimer free is subtracted from the absorption of the mixture of naphthyridine dimer and RRE.

Fig. 8 shows a UV absorption spectrum of a mixture of the naphthyridine-azaquinolone hybrid and RRE. In Fig. 8, the solid line indicates the UV absorption spectrum of RRE alone, and the dotted line indicates a UV absorption spectrum when the absorption of the naphthyridine-azaquinolone hybrid free is subtracted from the absorption of the mixture of the naphthyridine-azaquinolone hybrid and RRE.

Fig. 9 shows a UV absorption spectrum of a mixture the first quinoline-containing compound and RRE. In Fig. 9, the solid line indicates the UV absorption spectrum of RRE alone, and the dotted line indicates a UV absorption spectrum when the absorption of the first quinoline-containing compound free is subtracted from the absorption of the mixture of the first quinoline-containing compound and RRE. As represented by structural formula (7), the first quinoline-containing compound is obtained by replacing one naphthyridine of naphthyridine dimer with a quinoline incapable of hydrogen-binding to a guanine.

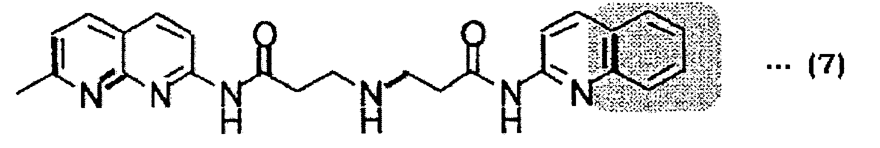

Fig. 10 shows a UV absorption spectrum of a mixture of a second quinoline-containing compound and RRE. In Fig. 10, the solid line indicates the UV absorption spectrum of RRE alone, and the dotted line indicates a UV absorption spectrum when the absorption of the second quinoline-containing compound free is subtracted from the absorption of the mixture of the second quinoline-containing compound and RRE. As represented by structural formula (8), the second quinoline-containing compound is obtained by replacing one naphthyridine of naphthyridine dimer with a quinoline incapable of hydrogen-binding to a guanine.

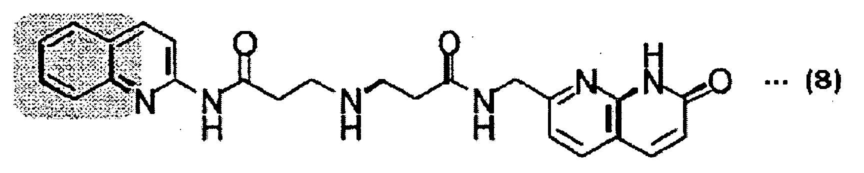

In the present example, comparison was made within a range of 300 nm to 400 nm where no absorption of RRE occurs.

Figs. 7 to 10 shows that there was significant changes in the UV absorption spectra when naphthyridine dimer capable of recognizing a GG mismatch and the naphthyridine-azaquinolone hybrid capable of recognizing a GA mismatch were used and, however, that there were no remarkable changes when the first and second quinoline-containing compounds were used, each of which contains quinoline in replacement of one naphthyridine ring. The quinoline is incapable of form hydrogen bond with guanine.

Naphthyridine is represented by structural formula (9). Azaquinolone is represented by structural formula (10). Quinolone is represented by structural formula (11). 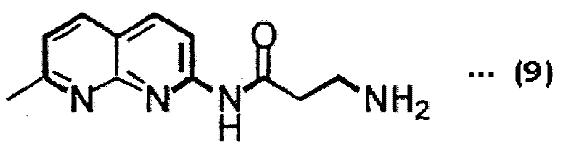 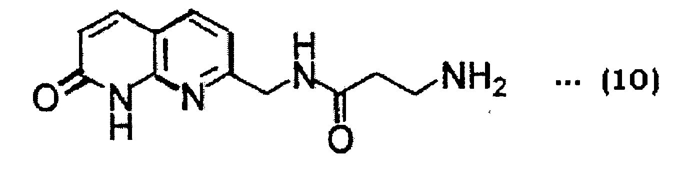 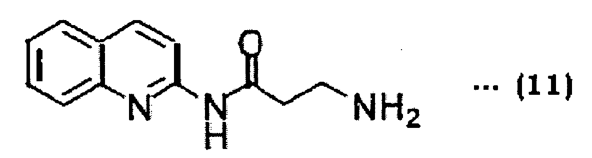 Comparing absorption of naphthyridine, azaquinolone, or quinolone alone and a mixture of naphthyridine, azaquinolone, or quinolone with RRE, there were almost no changes in a range of 300 nm to 400 nm. Figs. 11-13 respectively illustrate the UV absorption spectra of naphthyridine, azaquinolone, or quinolone alone and the mixture thereof with RRE.

These results showed that constituent molecules of naphthyridine dimer, which includes two naphthyridines, or the naphthyridine-azaquinolone hybrid, which has azaquinolone in replacement of one of the two naphthyridine of naphthyridine dimer, cannot bind with RRE when they are alone, and it is necessary for the constituent molecules to be in the form of naphthyridine dimer or the naphthyridine-azaquinolone hybrid to bind with RRE.

### [Example 3]

A change in CD spectrum of RRE due to its binding to each of the drugs was analyzed. The result of the analysis is shown in Fig. 14.

In Fig. 14, the solid line indicates a CD spectrum of RRE alone, the dotted line indicates a CD spectrum of a mixture of naphthyridine dimer and RRE, and the dashed line indicates a CD spectrum of a mixture of the naphthyridine-azaquinolone hybrid and RRE.

As shown in Fig. 14, there was a significant change in the whole spectrum when naphthyridine dimer was added to RRE. Further, there was a change at 300 nm or above also when the naphthyridine-azaquinolone hybrid was added to RRE.

Further, a change in CD spectrum of RRE-neg due to its binding to each of the drugs was analyzed. The result of the analysis is shown in Fig. 15. RRE-neg has no internal loop found in RRE, the internal loop including a GG mismatch and a GA mismatch. RRE-neg has a base sequence shown in Fig. 16.

The results shown Figs. 14 and 15 showed that each of naphthyridine dimer and the naphthyridine-azaquinolone hybrid specifically binds to the internal loop of RRE.

### [Example 4]

n a manner shown in Fig. 17, a binding strength of each of RRE, RRE-neg, TAR, and single-stranded RNA (ss RNA) with respect to naphthyridine dimer immobilized on an SPR sensor chip was analyzed. Like RRE, TAR is known as an HIV-RNA target. The result of the analysis is shown in Fig. 18. In Fig. 18, the solid line indicates the binding strength of RRE, the dotted line the binding strength of RRE-neg, the dashed line the binding strength of TAR, and the double-dashed line the binding strength of ssRNA. (A base sequence of TAR is shown in Fig. 19, and a base sequence of ssRNA is shown in Fig. 20.)

As shown in Fig. 18, a strong signal was obtained when RRE was used. This showed that the binding of RRE to naphthyridine dimer was strong. However, there were almost no responses in the other types of RNA. Particularly, although TARRNA has an internal loop as RRE does, TARRNA did not bind at all to naphthyridine dimer.

The result showed that naphthyridine dimer recognizes a base sequence of the internal loop of RRE.

### [Example 5]

UV titration was carried out in order to study how naphthyridine dimer bound to RRE. The result is shown in Figs. 21 and 22. Fig. 21 shows UV absorption spectra of naphthyridine dimer in the presence of RRE of various concentrations. Fig. 22 shows a proportion of naphthyridine dimer binding to RRE to the whole naphthyridine dimer added to RRE, the proportion being calculated from an absorption intensity at 332 nm of Fig. 21.

It was confirmed that as the concentration of RRE increased, the UV absorbance of naphthyridine dimer decreased and the UV absorbance of a complex increased above an isosbestic point.

This showed that naphthyridine dimer was intercalated into a duplex of RRE and that two naphthyridine dimers formed the complex.

### [Example 6]

By using a fluorescence polarization method, it was tested whether Rev-RRE complex formation was inhibited by naphthyridine dimer and the naphthyridine-azaquinolone hybrid.

A greater molecular weight results in a greater polarization degree, i.e., a greater anisotropy. As shown in Fig. 23, for a complex of (a) a Rev peptide (hereinafter abbreviated as "Rev-Fl") fluorescent labeled with fluorescein and (b) RRE, the molecular weight increases, and the anisotropy increases accordingly. The Rev-Fl has a structure shown in Fig. 24.

The anisotropy of the Rev-Fl in the presence of RRE of various concentrations was measured. The measurement gave a binding curve shown in Fig. 25, whereby the complex formation was confirmed.

Further, the binding curve thus obtained was analyzed. The analysis showed that the Rev-RRE dissociation constant was 16 nM.

### [Example 7]

Naphthyridine dimer, the naphthyridine-azaquinolone hybrid, and neomycine B were used as inhibitors, and anisotropies of each of the inhibitors was measured. The results of the measurement are shown in Fig. 26. Fig. 26 shows, in a descending order of anisotropic values, a dissociation curve of the case where the neomycine B was added to RRE, a dissociation curve of the case where the naphthyridine-azaquinolone hybrid was added to RRE, and a dissociation curve of the case where naphthyridine dimer was added to RRE. The horizontal axis indicates the concentration of each of the inhibitors.

Fig. 26 shows that the decreases of the anisotropy were observed at lower concentrations in the use of naphthyridine dimer or naphthyridine-azaquinolone hybrid than in the use of neomycine B. This showed that naphthyridine dimer and the naphthyridine-azaquinolone hybrid have higher potency of inhibiting Rev-RRE complex formation than neomycine B.

Further, the neomycine had an IC₅₀ value of 4 µM for inhibiting complex formation, naphthyridine dimer had an IC₅₀ value of 1.2 µM for inhibiting complex formation, and the naphthyridine-azaquinolone hybrid had an IC₅₀ value of 2 µM for inhibiting complex formation.

### [Example 8]

An IC₅₀ value of the trinaphthyridine-azaquinolone hybrid used as an inhibitor was obtained by using the fluorescence polarization method. The IC₅₀ value of the trinaphthyridine-azaquinolone hybrid was 0.2 µM. Further, in Fig. 27, the solid line indicates how a CD spectrum of RRE alone was changed, the dotted line indicates how a CD spectrum of RRE was changed when naphthyridine dimer was added to RRE, the dashed line indicates how a CD spectrum of RRE was changed when the trinaphthyridine-azaquinolone hybrid was added to RRE, and it is indicated how a CD spectrum of RRE was changed when the trinaphthyridine-azaquinolone hybrid was added to RRE.

Naphthyridine dimer, the naphthyridine-azaquinolone hybrid, the neomycine B, and the trinaphthyridine-azaquinolone hybrid were used as inhibitors, and anisotropies of each of the inhibitors was measured. The results of the measurement are shown in Fig. 28. (Fig. 28 shows, in a descending order of anisotropic values, a dissociation curve when neomycine B was added to RRE, a dissociation curve of the case where the naphthyridine-azaquinolone hybrid was added to RRE, a dissociation curve of the case where naphthyridine dimer was added to RRE, and a dissociation curve of the case where the trinaphthyridine-azaquinolone hybrid was added to RRE. The horizontal axis indicates the concentration of each of the inhibitors.) These results showed that the inhibitor containing the trinaphthyridine-azaquinolone hybrid is approximately twenty times as active as the neomycine B.

As described above, an inhibitor according to the present invention is an inhibitor for inhibiting binding of RRE to Rev protein. The inhibitor is arranged so as to contain naphthyridine dimer represented by general formula (1).

Further, the inhibitor according to the present invention is arranged so as to contain the naphthyridine-azaquinolone hybrid represented by general formula (2).

Further, the inhibitor according to the present invention is arranged so as to contain the trinaphthyridine-azaquinolone hybrid represented by general formula (3).

Further, the inhibitor according to the present invention is arranged so as to contain the trinaphthyridine-azaquinolone hybrid derivative represented by general formula (4).

This brings about an effect of providing an inhibitor which can inhibit binding of RRE to Rev protein by specifically binding to a GG mismatch base pair and/or a GA mismatch base pair in an internal loop of RRE.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

### INDUSTRIAL APPLICABILITY

An inhibitor of the present invention can be used as an anti-AIDS medicine which suppresses development of AIDS. Further, a naphthyridine derivative of the present invention can be used as a component of the foregoing inhibitor. Further, a naphthyridine-dimer-containing aldehyde and a naphthyridine-azaquinolone-hybrid-containing aldehyde of the present invention can be used as intermediates during production of a trinaphthyridine-azaquinolone hybrid and a derivative thereof. Further, a substrate and a sensor chip of the present invention can be used to arrange an instrument, such as a chip, a cuvette, or a plate, which has a function of detecting RRE contained in a culture medium or in a blood fluid.

## Claims

1. An inhibitor for inhibiting binding of RRE to Rev protein, the inhibitor comprising a naphthyridine derivative represented by general formula (A): 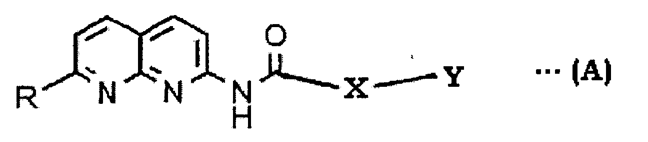 where R and R₁ are mutually independently a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms respectively substituted by an oxygen atom or a nitrogen atom, or a mono- or di-alkylamino group of a carbon number of 1 to 15, which is unsubstituted or has one or more carbon atoms substituted respectively by an oxygen atom or a nitrogen atom; X is an arbitrary bivalent group; and Y is a substituent represented by general formula (B) or a substituent represented by general formula (C):

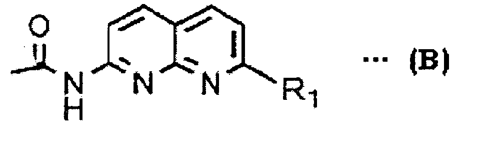

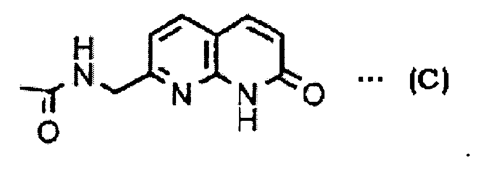

2. The inhibitor according to Claim 1, wherein the naphthyridine derivative is naphthyridine dimer represented by general formula (1): 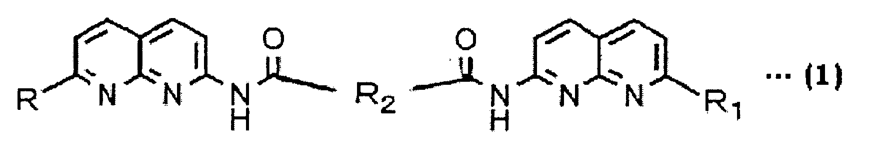 where R and R₁ are mutually independently a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom, and R₂ is an alkylene group of a carbon number of 1 to 20, which is unsbstituted or has one or more carbon atoms respectively substituted by an oxygen atom, a sulfur atom, a nitrogen atom, or a carbonyl group.

3. The inhibitor according to Claim 1, wherein the naphthyridine derivative is a naphthyridine-azaquinolone hybrid represented by general formula (2): 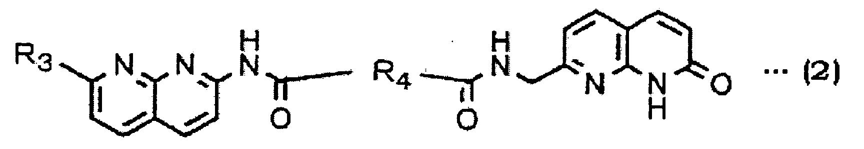 where R₃ is a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom, and R₄ is an alkylene group of a carbon number of 1 to 20, which is unsbstituted or has one or more carbon atoms respectively substituted by an oxygen atom, a sulfur atom, a nitrogen atom, or a carbonyl group.

4. The inhibitor according to Claim 1, wherein the naphthyridine derivative is a trinaphthyridine-azaquinolone hybrid represented by general formula (3): 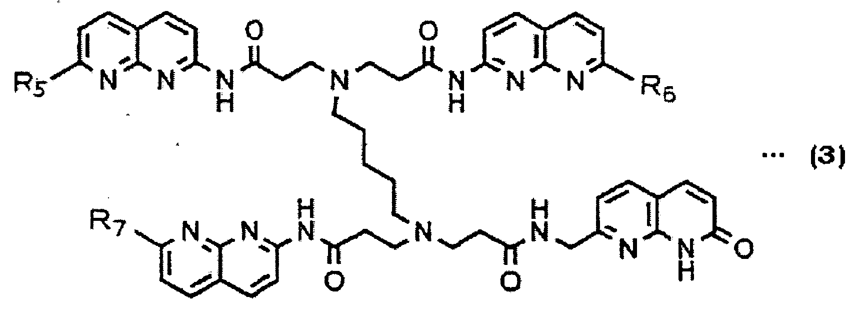 where R₅ ,R₆, and R₇ are mutually independently a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom.

5. The inhibitor according to Claim 1, wherein the naphthyridine derivative is a trinaphthyridine-azaquinolone hybrid derivative represented by general formula (4): 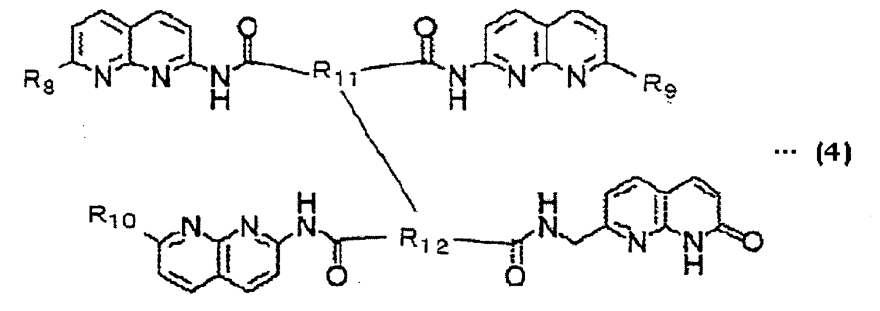 where R₈, R₉, and R₁₀ are mutually independently a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom, and R₁₁ and R₁₂ are mutually independently an alkylene group of a carbon number of 1 to 20, which is unsubstituted or has one or more carbon atoms substituted respectively by an oxygen atom, a sulfur atom, a nitrogen atom, or a carbonyl group.

6. A naphthyridine derivative serving as an inhibitor for inhibiting binding of RRE to Rev protein, the naphthyridine derivative being represented by general formula (A): 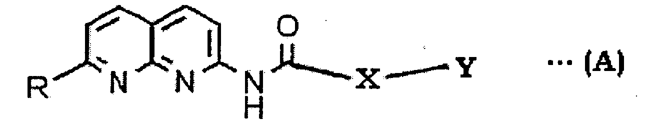 where R and R₁ are mutually independently a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom; X indicates a given bivalent group; and Y is a substituent represented by general formula (B) or a substituent represented by general formula (C).

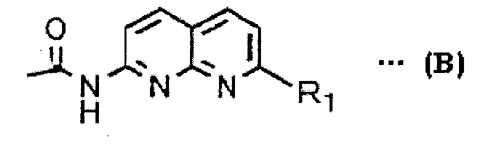

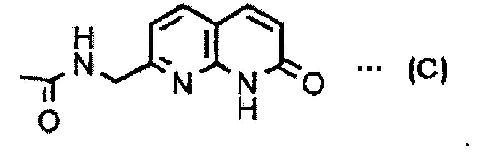

7. The naphthyridine derivative according to Claim 6 being a naphthyridine-dimer-containing aldehyde represented by general formula (5): 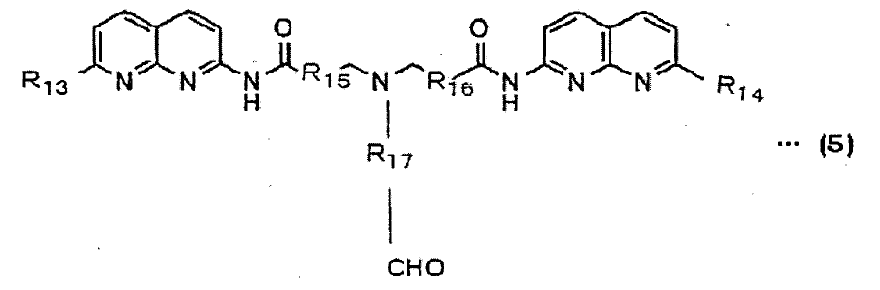 where R₁₃, and R₁₄ are mutually independently a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom; R₁₅ and R₁₆ are mutually independently an alkylene group of a carbon number of 1 to 18, which is unsubstituted or has one or more carbon atoms substituted respectively by an oxygen atom, a sulfur atom, a nitrogen atom, or a carbonyl group; and R₁₇ is an alkylene group of a carbon number of 1 to 14, which is unsubstituted or has one or more carbon atoms substituted respectively by an oxygen atom or a nitrogen atom.

8. The naphthyridine derivative according to Claim 7 obtained by allowing naphthyridine dimer to react with a di-aldehyde represented by general formula (D):
OHC-R_{A}-CHO (D)
where R_{A} is an alkylene group of a carbon number of 1 to 13, which is unsubstituted or has one or more carbon atoms substituted respectively by an oxygen atom or a nitrogen atom.

9. The naphthyridine derivative according to Claim 6 being a naphthyridine-azaquinolone-hybrid-containing aldehyde represented by general formula (6): 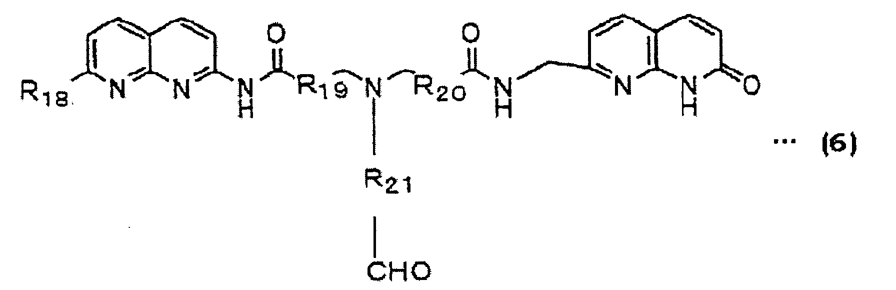 where R₁₈ is a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom; R₁₉ and R₂₀ are mutually independently an alkylene group of a carbon number of 1 to 18, which is unsubstituted or has one or more carbon atoms substituted respectively by an oxygen atom, a sulfur atom, a nitrogen atom, or a carbonyl group; and R₂₁ is an alkylene group of a carbon number of 1 to 14, which is unsubstituted or has one or more carbon atoms substituted respectively by an oxygen atom or a nitrogen atom.

10. The naphthyridine derivative according to Claim 9 obtained by allowing a naphthyridine-azaquinolone hybrid to react with a di-aldehyde represented by general formula (D):
OHC-R_{A}-CHO (D)
where R_{A} is an alkylene group of a carbon number of 1 to 13, which is unsubstituted or has one or more carbon atoms substituted respectively by an oxygen atom or a nitrogen atom.

11. The naphthyridine derivative according to Claim 6 being naphthyridine dimer represented by general formula (1): 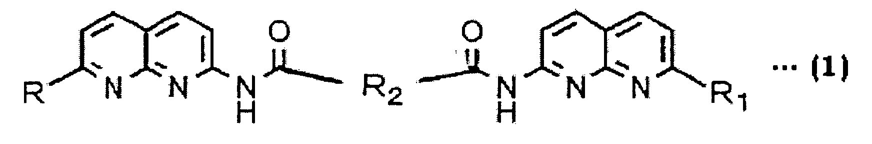 where R and R₁ is a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom, and R₂ is an alkylene group of a carbon number of 1 to 20, which is unsubstituted or has one or more carbon atoms substituted respectively by an oxygen atom, a sulfur atom, a nitrogen atom, or a carbonyl group.

12. The naphthyridine derivative according to Claim 6 being a naphthyridine-azaquinolone hybrid represented by general formula (2): 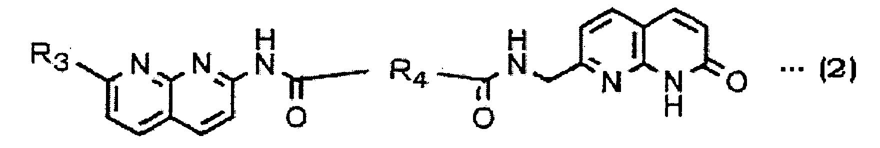 where R₃ is a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom, and R₄ is an alkylene group of a carbon number of 1 to 20, which is unsubstituted or has one or more carbon atoms substituted respectively by an oxygen atom, a sulfur atom, a nitrogen atom, or a carbonyl group.

13. The naphthyridine derivative according to Claim 6 being a trinaphthyridine-azaquinolone hybrid represented by general formula (3): 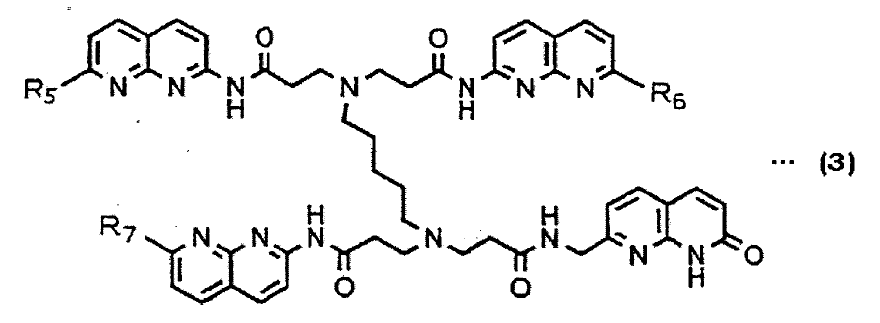 where R₅ ,R₆, and R₇ are mutually independently a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom.

14. The naphthyridine derivative according to Claim 6 being a trinaphthyridine-azaquinolone hybrid derivative represented by general formula (4): 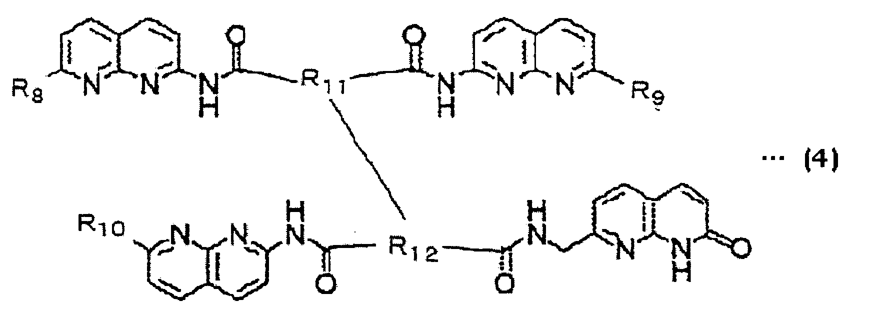 where R₈, R₉, and R₁₀ are mutually independently a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom, and R₁₁ and R₁₂ are mutually independently an alkylene group of a carbon number of 1 to 20, which is unsubstituted or has one or more carbon atoms substituted respectively by an oxygen atom, a sulfur atom, a nitrogen atom, or a carbonyl group.

15. A substrate comprising a composite which contains at least one compound selected from the compound group consisting of naphthyridine dimer, a naphthyridine-azaquinolone hybrid, and a trinaphthyridine-azaquinolone hybrid or a derivative thereof.

16. A biosensor for detecting RRE, the biosensor including a carrier on which a naphthyridine derivative is immobilized, the naphthyridine derivative being represented by general formula (A): 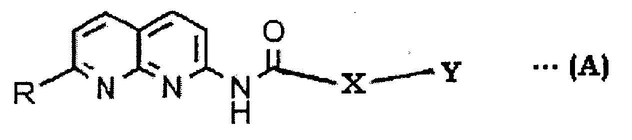 where R and R₁ are mutually independently a hydrogen atom, an alkyl group of a carbon number of 1 to 15, which is unsubstituted or has one or more carbon atoms substituted respectively by an oxygen atom or a nitrogen atom, an alkoxy group of a carbon number of 1 to 15, which is unsubstituted or has one or more carbon atoms substituted respectively by an oxygen atom or a nitrogen atom, or a mono- or di-alkylamino group of a carbon number of 1 to 15, which is unsubstituted or has one or more carbon atoms substituted respectively by an oxygen atom or a nitrogen atom; X is a given bivalent group; and Y is a substituent represented by general formula (B) or a substituent represented by general formula (C).
